# EUROPEAN PATENT APPLICATION

(11) **EP 4 023 352 A1**
(43) Date of publication of application: **06.07.2022**
(21) Application number: 21212403.6
(22) Date of filing: 03.12.2021
(51) Int. Cl.: B09B 3/00, B02C 18/00, B02C 18/12, B02C 18/22, B09B 3/40

(54) **MACHINE FOR THE PROCESSING OF WASTE AND RELATED STORAGE METHOD**

(30) Priority: 07.12.2020 IT 202000030113; 07.12.2020 IT 202000030110
(71) Applicant: Ompeco S.r.l., 10051 Avigliana (TO) (IT)
(72) Inventor: SIMONE, Matteo, 10051 Avigliana (Torino) (IT); LOMBARDO, Massimo, 10051 Avigliana (Torino) (IT)
(74) Representative: De Bonis, Paolo

(57) **Abstract**

Disclosed herein is a machine (1) for the processing of waste, including
- a processing cell (2) configured to receive waste to undergo processing in a processing volume (V2), the processing cell (V2) comprising a main axis (Z2), a closable loading opening (4) which is located at a top end (2T) and a closable discharge opening (5) which is located at a bottom end (2B) of said processing cell, the bottom end (2B) and the top end (2T) being opposite ends along said main axis (Z2),
- a rotor (7) located in the processing volume (V2) of said processing cell (2) at the bottom end (2B) thereof, the rotor (Z2) being operable in rotation around said main axis (Z2) and comprising cutting elements configured to heat and shred the waste in said processing volume (V2).

The machine includes a storage unit, adapted to operate with different operation and storage modes, also including vacuum storage.

## Description

### Field of the Invention

The present invention refers to machines for the processing of waste, specifically to machines for cooking and stabilizing bio-organic and other waste. An example of such machines is disclosed in European Patent n. EP 1 546 623 B1.

### State of the Art

The machines for the processing of waste to which the present invention refers are adapted to cook, through heat and locally generated steam, biologic material such as biomass, waste from food processing and, more generally, materials which contain or may contain biologic substances, including living organisms. Moreover, it is possible to process inorganic and/or non-biologic waste, such as plastics and metals. Such processing stabilizes the materials in the form of a sterilized and dehydrated product, which is stable for long periods.

Such machines generally include a processing cell, having a cylindrical processing volume including a loading opening located at the top and a discharge opening located near the bottom. The cell has a generally vertical axis, and around said axis a rotor is rotatably mounted and operates within the processing cell, typically near the bottom of the cell itself. The rotor advantageously comprises cutting profiles, which are adapted to heat and shred the waste processed within the processing cell, wherein heating and shredding take place thanks to the combination of the friction between the rotor and the waste and of the dissipation of the kinetic energy when the rotor cutting profiles impinge onto the waste. In some embodiments, such as described in EP 1 546 623 B1, the heating action is magnified and optimized by the presence of fixed profiles, adapted to cooperate with the cutting profiles on the rotor. The repeated interaction between the rotor and the waste causes the overheating of the latter, with a consequent evaporation of the waste liquid fraction. After overheating, the machine is adapted to administer a controlled amount of water, so as to decrease temperature and achieve devitalization of the bio-organic material which may be present in the waste.

Such machines are generally available on the market in a range of sizes, which depend on the capacity (volume) of the processing cell. This implies that the machines of the same range differ from one another essentially for their size, with the exception of a few dimensions which change according to structural strength requirements (e.g. the hub and the spokes of the rotor, or the cutting inserts on the rotor itself), which may lead to local modifications of the aspect ratio of the machine in the various sections thereof. This may alter the performance of the machine as a function of the size, especially in low-capacity machines. In such machines, the interaction between the rotor, the processing cell and the waste may be less efficient, especially in the machines wherein only the rotor is provided with cutting profiles adapted to shred the waste. Especially small-capacity machines may offer a worse mixing ratio, due to an unfavourable aspect ratio between the cell and the rotor.

A further technical problem observed in the known machines for the processing of waste refers to the kind of storage of the processed waste. Known machines are only adapted to expel the processed waste, while leaving it up to the user the task of storing waste in containment units which may ensure easy handling and a long lasting preservation according to regulations.

### Object of the Invention

The invention aims at solving the technical problems mentioned in the foregoing. Specifically, the invention aims at providing a machine for the processing of waste which implements a safe and automatic storage of the processed waste. A further object of the invention is providing the storage of waste in different conditions, without requiring additional equipment for the processing machine.

Another object of the invention is providing a machine for the processing of waste with improved performance, having a high mixing ratio even in low-capacity models.

### Summary of the Invention

The object of the invention is achieved thanks to a machine for the processing of waste having the features set forth in the Claims that follow, and which are an integral part of the technical teaching provided herein with reference to the invention.

### Brief Description of the Figures

The invention will now be described with reference to the annexed Figures, which are provided by way of example only and wherein:
- Figure 1 is an overall view of a machine for the processing of waste according to the invention,
- Figure 2 is a cross-section view along line II-II in Figure 1,
- Figure 3 is an isolated perspective view of a processing cell of the machine according to the invention,
- Figure 4 is a cross-section view along line IV-IV in Figure 3,
- Figure 5 is a detail according to arrow V of Figure 2, and
- Figure 6 is a view similar to Figure 5, but showing a further aspect of the present invention.

### Detailed Description

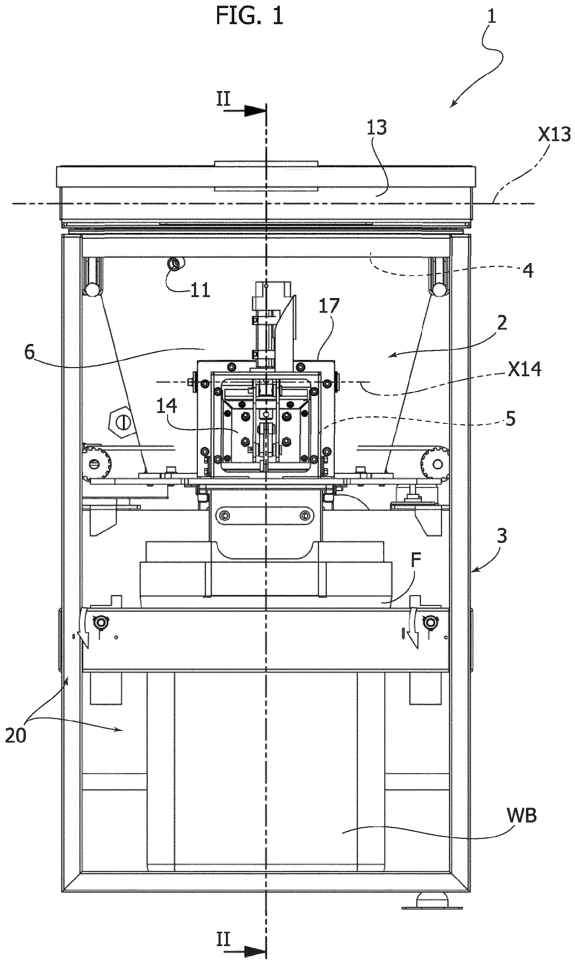

Reference 1 in Figure 1 generally denotes a machine for the processing of waste according to the invention. With reference to Figures 1 to 4, in various preferred embodiments, the machine 1 includes a processing cell 2, adapted to receive waste to be processed (specifically in a cooking and stabilizing processing) in a processing volume V2, and it has a main axis Z2 arranged vertically. The processing cell 2 is installed on a frame 3 carrying all the further components of the machine 1, and it includes a closable loading opening 4 which is located at a top end 2T and an equally closable discharge opening 5 which is arranged at a bottom end 2B, wherein the bottom end 2B and the top end 2T are opposite ends 2 along the main axis Z2.

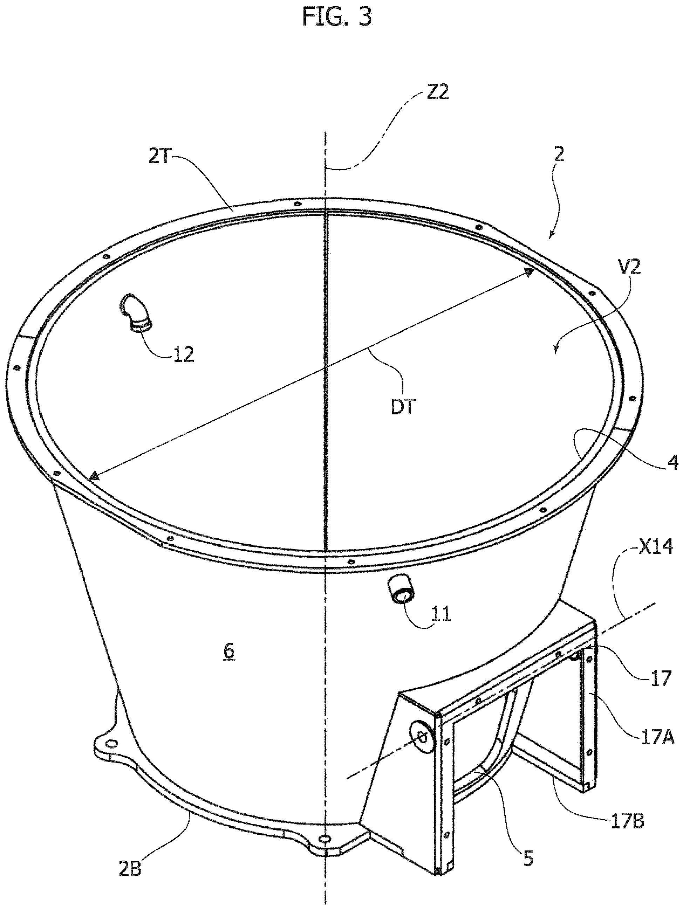

Referring to Figure 3, the processing volume V2 has an inner diameter extending transversely to the main axis Z2 and decreasing along said main axis Z2 towards the bottom end 2B.

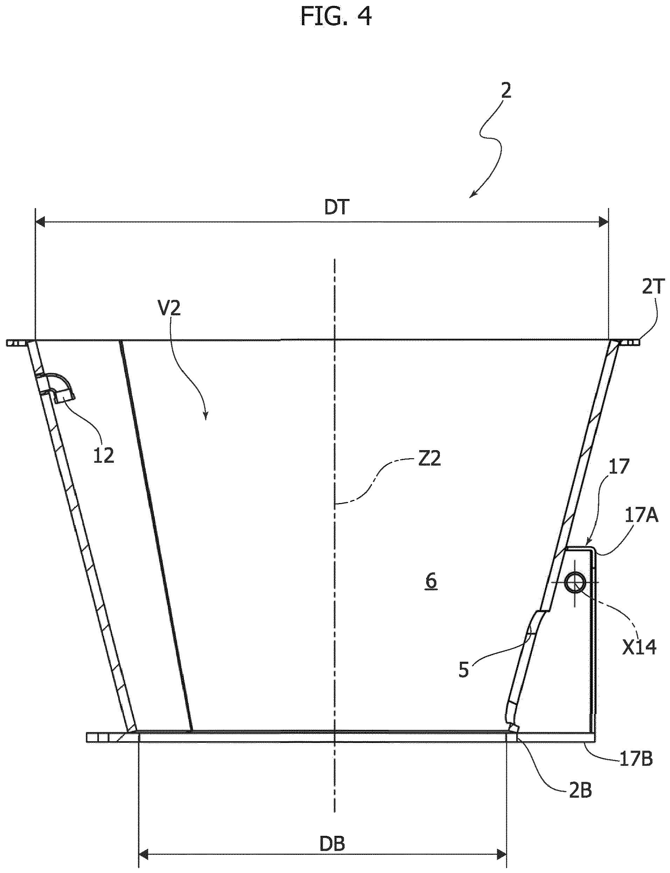

With reference to Figures 3, 4, in a preferred embodiment the internal volume V2 includes an inner diameter having a first value DT at the top end 2T of the processing cell 2, and an inner diameter having a second value DB at the bottom end 2B of the processing cell. According to the invention, diameter DT is longer than diameter DB, and the inner diameter decreases from the first value DT to the second value DB along the main axis Z2 from the top end 2T to the bottom end 2B.

In various embodiments, including the embodiment shown in the Figures, the inner diameter of the processing volume V2 decreases at a constant rate. In other words, the processing volume V2 (and in the present case the overall cell 2 as well) includes a conical wall 6 the diameters whereof decrease from diameter DT to diameter DB in direct proportion to the position along the axis Z2, i.e. in direct proportion to the axial distance from diameter DT.

Specifically, preferred values of the reduction coefficient of the diameter as a function of the axial position are comprised between 0.2 and 0.3.

In other embodiments, the inner diameter of the processing volume V2 decreases from the first value DT to the second value DB at a variable rate. In other words, the processing volume V2 includes a wall the diameters whereof decrease from diameter DT to diameter DB without following a direct proportion relation between the axial position of the inner diameter and the value thereof.

In still further embodiments, the inner diameter may decrease towards the bottom end 2B for a part of the extension of the processing volume V2 along axis Z2. The reduction may have either a constant or a variable rate, and the portion(s) which are not involved in the reduction may have either a constant inner diameter or an inner diameter which increases from the top end 2T towards the bottom end 2B; of course, such portions (or such a portion) will necessarily be located above, or anyway closer to end 2T than, the portion exhibiting a decrease towards bottom end 2B.

Within the processing cell 2, specifically within the processing volume V2 and at the bottom end 2B (and generally at the bottom of the cell), there is provided a rotor 7, so that the inner diameter of the processing volume V2 decreases towards the rotor 7. Rotor 7 is rotatably mounted around axis Z2, and to this end it is operable in rotation by an electrically-driven motor (or, in some applications, by a gear motor assembly) 8. The motor (or gear motor) 8 is carried by frame 3.

In a way per se known, the rotor 7 includes at least two spokes (typically two, but in certain applications the number thereof may be higher) which are provided with cutting and/or shredding inserts. In an embodiment, the rotor includes a pair of wedge-shaped cutting inserts at the leading edge of each of the spokes; the leading edge is the edge which first contacts the waste during the rotation of rotor 7. In other embodiments, inserts may be provided both on the leading edge and on the trailing edge, in order to allow the rotor to shred and heat the material in both rotation directions (clockwise and anti-clockwise). Moreover, in some embodiments the cutting inserts on the rotor are configured to cooperate with fixed wedge-shaped profiles or with fixed blades arranged in a circular crown on the wall 6 of processing volume V2 (whatever the geometry thereof may be), and/or on the bottom of the cell itself. An example of such a structure is described in EP 1 546 623 B1.

The machine 1 moreover comprises:
- a negative pressure fluidic circuit which is in fluid communication with the internal volume V2 of the processing cell 2, and which is controllable via a supply unit adapted to establish a negative pressure within the processing volume V2, wherein the supply unit preferably includes a vacuum pump 9 (e.g. a liquid ring pump), and
- a circuit for dosing water for devitalization and temperature control, which is preferably supplied with water from the mains, in fluid communication with the volume V2 of the cell 2.

In more detail, the circuit for dosing water for devitalization and temperature control is in fluid communication with the volume V2 through a nozzle 12 located close to the loading opening 4, so as to impinge on the waste from above downwards.

In order to perform the processing of waste in the volume 2 at sub-atmospheric pressures, the loading opening 4 and the discharge opening 5 may be hermetically sealed respectively by a cover 13 and by a hatch 14. Both the cover 13 and the hatch 14 may be actuated between a closing position, wherein they are respectively in abutment on the openings 4 and 5, therefore ensuring a fluid-tight seal thereat, and an opening position, wherein they are raised or in any case away from the respective opening, so as to enable the flow of material.

In this respect, the negative pressure fluid circuit is in fluid communication with volume V2 of processing cell 2 via the cover 13, which carries a suction opening (which is preferably shielded by a grate, in order to prevent big-sized debris from entering the negative pressure circuit) which is in fluid communication with the volume V2, so as to enable venting the air present inside.

The cover 13 and the hatch 14 are actuated by respective electro-mechanical actuators around axes which are transverse to axis Z2 and are denoted by references X13 (for cover 13) and X14 (for hatch 14). The actuator which drives the hatch 14 is denoted by reference 16, while the actuator which drives the cover 13 is not visible in the Figure (in a preferred embodiment, the actuator is a gas spring).

Referring specifically to Figure 3, the processing cell 2 advantageously includes an interface chamber 17 which communicates with the discharge opening 5 and is configured to house the hatch 14, which is pivotally mounted on a pair of bushings, located on the walls of chamber 17, which define the axis X14. The chamber 17 is open at the front, directly facing the opening 5, and at the bottom, so as to implement two connection interfaces 17A (on the front), 17B (on the bottom) and other components of the machine 1 which will be described in detail in the following.

The front connection interface 17A receives a pan 18 on which the actuator 16 is installed, and moreover acts as an additional containment housing for the hatch 14 and the actuating mechanism thereof. On the other hand, the bottom interface 17B receives a drop channel 19 (so that the interface chamber globally communicates with the discharge opening 5 and with the channel 19), configured to convey the processed waste coming from the opening 5 into a storage unit 20, which defines another advantageous feature of the invention.

At the interfaces 17A and 17B, as well as at the interfaces between the pan 18 and the drop channel 19, fluid-tight elements are advantageously provided, such as film or bead gaskets, in order to guarantee a hermetical sealing of the opening 4 against the environment.

It will be observed, moreover, that the storage unit 20 may be applied to any machine for the processing of waste similar to machine 1, irrespective of the structure of the cell 2, specifically irrespective of the configuration of the volume V2 having either a constant or a variable volume, and irrespective of the structure of the chamber 17.

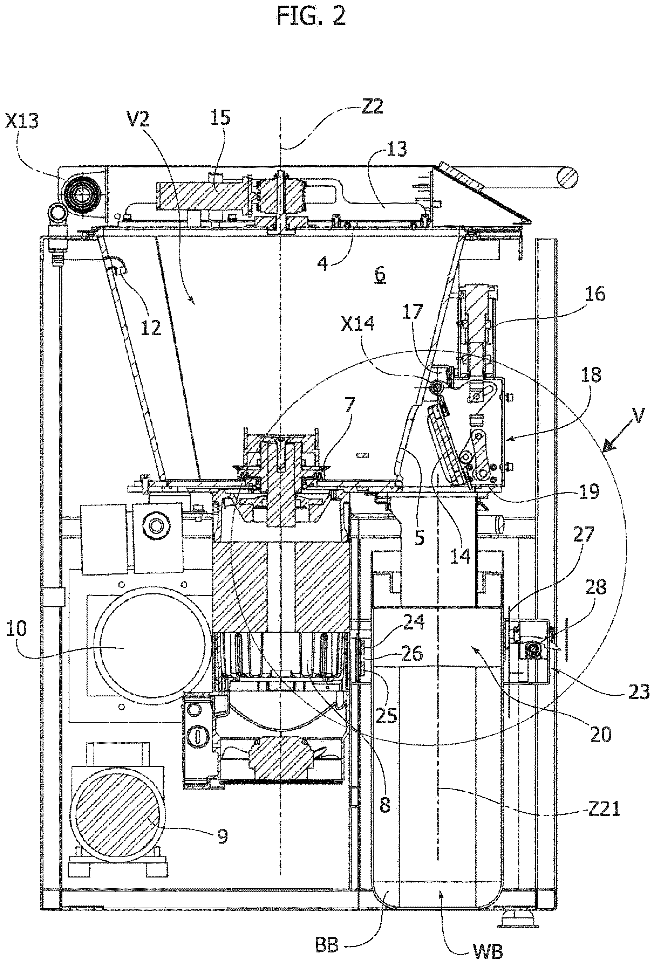

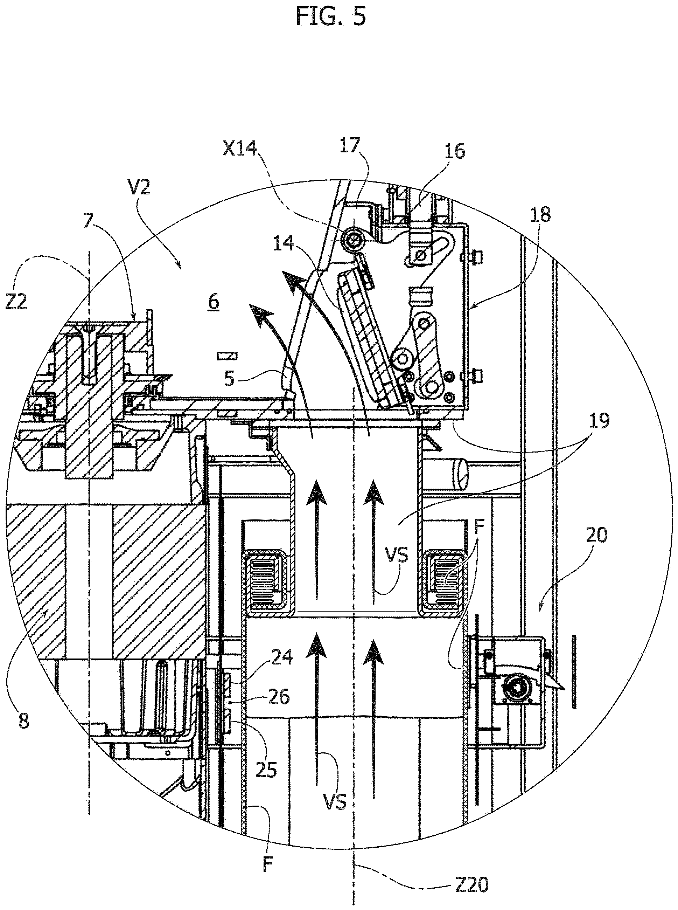

Referring to Figure 2 and Figure 5, the storage unit 20 includes a annular support 21, adapted to receive a sleeve of film material F, which is stored in a collapsed configuration and which may be deployed in order to define a receiving lumen for the waste processed by machine 1, wherein the receiving lumen may be sealed in order to define a bag WB which will receive the waste processed by the machine 1. In operation, a part of the sleeve F is stored in a collapsed configuration, and a part is deployed in order to form the bags WB until finishing the storage.

In the preferred embodiment illustrated herein, the sleeve F is accumulated in a annular groove 22, and it is folded inwardly so as to be guided into the ring-shape support 21. There is provided a further fold, which leads the sleeve F to exit support 21, to wrap around the annular groove 22 and to align again with direction Z21, so as to be in line with the drop channel 19. Preferably, the drop channel 19 is arranged coaxially to the axis Z1, but more generally it is sufficient if the internal lumen of the channel 19 leads into the annular support, so as to be contained within the receiving lumen defined by the sleeve F on the annular support 21.

The receiving lumen of the sleeve F may be sealed by a closing unit 23, having one or more closing devices configured to seal the receiving lumen at a bag bottom and at a bag head in order to define bag WB, and further comprising a separation device 26, configured to separate a bag head of a first bag WB from the bag bottom of a second bag WB following the first bag along the sleeve of film material F.

In the preferred embodiment illustrated herein, the closing unit 23 includes a first sealing head 24, a second sealing head 25 and a cutting device 26 (separation device). In the present embodiment, the three elements 24, 25, 26 cooperate with a sealing bar 27, which is movable transversely to the axis Z21 and which is operated in translation by an actuator 28.

Specifically, the heads 24, 25 and the cutting device 26 are preferably of the thermal type, and they are configured to pinch the receiving lumen of the sleeve F when the transverse bar 27 is approached thereto. The heads 24 and 25 form a pair of thermal welds which respectively define a bag bottom and a bag head. On the other hand, the cutting device 26 is adapted to heat the portion of the sleeve F between the heads 24 and 25, until this leads to severing, in order to separate two subsequent bags WB. As an alternative, the heads 24, 25 and the cutting device 26 may be replaced by ultrasonic elements.

The machine 1 operates as follows.

The machine 1 functionally operates as a machine for the processing (shredding, cooking and stabilizing) of waste of a known type. The waste to be processed, whatever the nature thereof, is loaded into the volume V2 of the processing cell 2 through the loading opening 4, which is made accessible to this end by raising the cover 13 through the respective actuator. The discharge opening 5 is kept closed by the hatch 14.

Once the waste has been loaded into the volume V2, the opening 4 is closed by applying and hermetically sealing the cover 13, and the working cycle is started. First of all, this provides for the establishment of a negative pressure within the volume V2, by activating the vacuum pump 9 which, through the fluid connection with the cover 13, sucks away the air contained in the volume V2 through the intake port located on the cover 13 itself.

Thereafter, the rotation of the rotor 7 is started: the interaction between the waste and the rotor 7 rotating around axis Z2 causes the shredding and the overheating the waste within the volume V2, with temperatures that may reach as high as 150° C or even 180° C. During the interaction between the rotor 7 and the waste, the walls of the processing volume V2 of the cell 2, which by virtue of the foregoing description have a diameter which decreases towards the rotor 7, naturally convey the waste towards the rotor 7 with a mixing rate which is higher than in the known machines, moreover increasing the energy transfer from the rotor 7 to the waste by exerting a slight compression of the mass that is processed towards the rotor 7 due to the tapering of the wall 6. This increases the overall efficiency of the processing and of the machine 1 and, especially in low-capacity sizes, the inefficiencies of the prior art machines are eliminated. As previously described, low-capacity machines may exhibit the problem of a smaller area covered by the spokes of the rotor 7 with respect to the sectional area of the volume V2, because, due to structural requirements, the rotor hub cannot scaled down linearly as compared to larger sized machines. The small area covered by the spokes of rotor 7 is compensated for, - almost cancelling the effect thereof - by the tapering of the wall 6 of the cell towards the rotor 7: by increasing the mixing of the waste and by setting up a slight compression of the mass being processed towards the rotor 7, the machine 1 operates with a performance comparable to larger sized conventional machines.

When overheating is completed, the shredded waste mass is stabilized through the addition of a controlled amount of water via the nozzle 12. Preferably, the water flow rate is adjusted via a solenoid valve upstream of the nozzle 12 (or embedded within the nozzle 12), which as previously mentioned is preferably supplied with water from the water mains. In this way the waste, which has previously been sterilized and is substantially devoid of water, is stabilized, leading to devitalization of possible residual bio-organic components.

The processing of waste is thus completed: the rotor 7 is stopped and the hatch 14 is opened, thus opening the discharge opening 5. The rotor 7 is operated again for a short time, in order to expel the processed waste through the opening 5: from here, the waste is routed through a path traversing the chamber 17 and the drop channel 19, to then give out into the receiving lumen of the sleeve of film material F, which is already sealed at a bag bottom BB so as to form the bag WB.

When the bag WB is filled with the desired amount of waste, while keeping the cover 13 in the closed position and the hatch 14 in the open position, the vacuum pump 9 is actuated in order to suck air away from all the volumes, including volume V2, the volume of the chamber 17 and of the pan 18, the volume of the drop channel 19 and the internal volume of the bag WB. In this regard, Figure 5 shows oriented arrows VS, which mark the air path during air suction. Said air suction is made possible because the discharge opening 4 communicates with all above-mentioned volumes, and the chamber 17 has a fluid-tight configuration.

Thereafter, the closing unit 23 is operated, so as to define a bag head with the sealing head 25 and a bag bottom for the subsequent bag WB, which is downstream of the bag which has just been filled with respect to the process direction (top down direction in the Figures), and which is above the bag which has just been filled, with regard to the spatial arrangement thereof. The cutting device 26 completes the cycle by separating the bag head of the bag WB which has just been filled from the bag bottom of the new bag WB. Subsequently, the rotor 7 is actuated again, in order to fill as many bags WB as needed in order to contain the waste mass processed in volume V2, wherein, at the end of the filling of each bag WB, the vacuum pump 9 is actuated in order to suck the air away from bag WB before sealing it.

The person skilled in the art will appreciate that the working cycle of machine 1 ends with a product which is automatically stored in vacuum packed bags, for which the storage times are remarkably longer as compared with the traditional storage in a bag containing air or in a rigid container. It will be observed, moreover, that such an exceptional result is achieved without adding any equipment to the storage unit 20: the whole negative pressure circuit which enables achieving a vacuum storage in the bags WB consists of components already present in such machines, i.e. pump 9 and the related negative pressure circuit, because they are used to perform the normal processing of waste.

Nevertheless, it is possible to operate by relying on the automatic and continuous bagging without applying vacuum to the bags via the negative pressure circuit: this is a possibility offered by the invention which is particularly advantageous in case waste is processed for which vacuum storage does not provide significant advantages compared to atmospheric storage (e.g. metal or glass waste, and generally speaking waste of inorganic origin).

It will be observed, moreover, that thanks to a further advantageous feature of the invention, the storage unit 20 may be replaced, in the case of small processing volumes and/or few machine cycles per day, with a conventional storage unit including one single bag directly coupled to the drop channel 19, which receives the waste processed within the volume V2. Similarly to what has been described with reference to the operation of the machine 1 provided with the storage unit 20, in this embodiment the processing cycle and the discharge of waste into the single bag is identical, as is the air suction step from the bag volume at the end of the introduction of waste, which always takes place through the negative pressure circuit of the machine. The only difference consists in the fact that, once the air has been sucked away, the bag is sealed by a closing unit similar to the closing unit 23 (the assemblies may even be identical: the transversal sealing of a bag does not depend on the nature thereof, which may be either a pre-formed bag or a bag obtained via sequential welds of a continuous sleeve of material) and is simply removed from machine 1. The operator will then manually install a new bag for the subsequent working cycle.

Generally, the method according to this embodiment of the invention includes:
- coupling a waste storage bag onto the drop channel 19; in this regard, it will be observed that the drop channel 19 may be embodied as incorporating the annular support 21, in such a way as to be able to operate, with the same machine, with a continuous bagging (with or without vacuum)
- enabling the communication between the processing volume V2 and the drop channel 19 through the discharge opening 5,
- rotatably actuating the rotor 7 in order to send a desired amount of processed waste, through the discharge opening 5 and the drop channel 10, into the waste storage bag,
- actuating the supply unit 9 of the negative pressure fluid circuit, in order to suck air away from the waste storage bag,
- sealing the waste storage bag, e.g. with a closing unit similar to assembly 23.

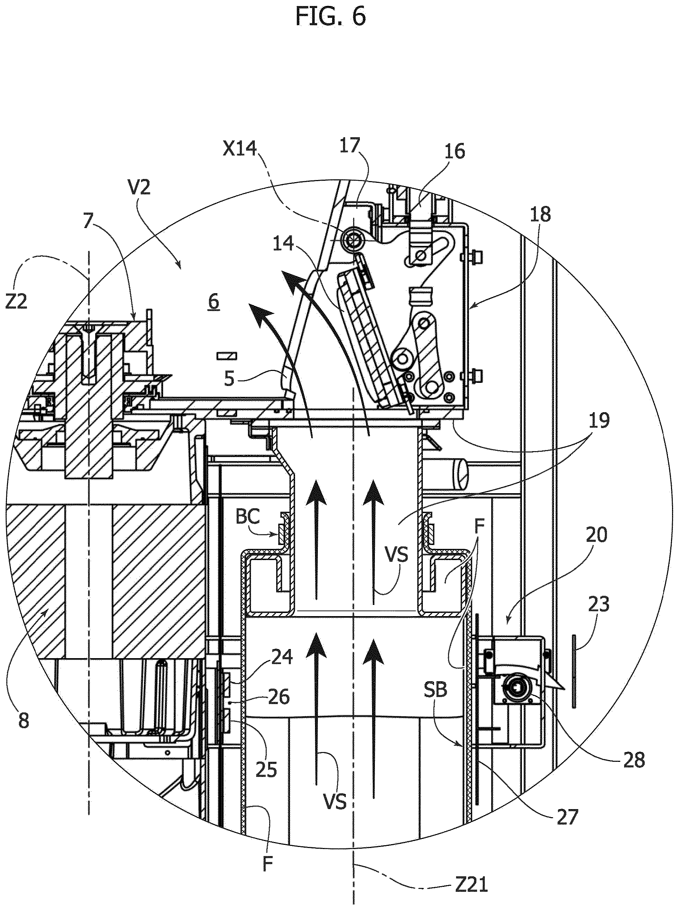

Moreover, referring to Figure 6, it is also possible to keep the same structure of the storage unit 20 of Figures 1, 2, 5, while eliminating only the sleeve of film material F, which enables a continuous production of bags of material, and coupling one single bag SB onto the annular support 21 which is used as an extension of the drop channel 19, sealing the bag may be achieved by the same closing unit 23 provided in machine 1. Specifically, Figure 6 shows a possible application of bag SB onto the annular support, which envisages the use of an annular band BC (e.g. an annular clamp, possibly of an elastomeric material to enhance the sealing effect), in order to press the inlet of the bag SB against the walls of drop channel 19 and to wrap the annular support 21. Also in this case oriented arrows VS are displayed, which illustrate the air flow when vacuum is applied to the bag SB through the cell 2 and the volume V2, the opening 5, the interface chamber 17 and all the volumes which are in fluid communications therewith.

Of course, what has been described in the foregoing may be applied irrespective of the geometry of the cell 2, because the shape (either conical, generally and/or partially tapered or cylindrical) of volume V2 does not exclude the possibility of applying a negative pressure for sucking air away from the bag SB or from the sleeve F, when the latter is sealed to form a bag WB.

Of course, the implementation details and the embodiments may vary, even appreciably, from what has been described and illustrated herein, without departing from the scope of the present invention, as defined in the annexed Claims.

## Claims

1. A machine (1) for waste processing, comprising:
- a processing cell (2) configured for receiving waste to undergo processing into a processing volume (V2) thereof, the processing cell (V2) comprising a main axis (Z2), a closable loading opening (4) arranged at a top end (2T) thereof, and a closable discharge opening (5) arranged at a bottom end (2B) thereof, the bottom end (2B) and the top end (2T) being opposite ends along said main axis (Z2),
- a rotor (7) arranged in the processing volume (V2) of said processing cell (2) at the bottom end (2B) thereof, the rotor (Z2) being operable in rotation around said main axis (Z2) and comprising cutting elements configured for heating and shredding the wastes in said processing volume (V2),
the machine (1) being **characterized in that** the discharge opening (4) is in communication (17, 19) with a storage unit (20), the storage unit (20) comprising:
- an annular support (21) configured to receive a sleeve of film material (F) partially accumulated in a collapsed configuration and deployable to define a receiving lumen for the waste processed by machine (1), said receiving lumen being closable to define a bag (WB) for containing the waste processed by the machine (1),
- a closing unit (23) comprising one or more closing devices (24, 25), configured to close the receiving lumen at a bag bottom and at a bag head to define said bag (WB), and further comprising a separation device (26) configured to separate a bag head of a first bag (WB) from the bag bottom of a second bag (WB) which follows the first bag along the sleeve of film material (F).

2. The machine (1) according to Claim 1, comprising an interface chamber (17) in communication with said discharge opening (5) and with a drop channel (19) which opening into the annular support (21) of the storage unit, so as to route the waste coming from said discharge opening (5) into the receiving lumen of the sleeve of film material (F).

3. The machine (1) according to Claim 1 or Claim 2, wherein said interface chamber (17) is hermetically sealed, and wherein the loading opening (4) and the discharge opening (5) are hermetically closable (13, 14) .

4. The machine (1) according to Claim 2 or Claim 3, further comprising a negative pressure fluidic circuit in fluid communication (11) with the processing volume (V2), the fluidic circuit being controllable by means of a supply unit (9) configured to establish a negative pressure within the processing volume (V2).

5. The machine (1) according to Claim 4, wherein the negative pressure fluidic circuit is in fluid communication with said interface chamber (17) when said discharge opening (4) is accessible.

6. A method for the storage of waste processed in a processing machine (1) according to any one of the preceding Claims, comprising:
- enabling a communication between the processing volume (V2) and the storage unit (20) through the discharge opening (5),
- rotatably actuating the rotor (7) to direct a desired amount of processed waste through said discharge opening (5) and into the receiving lumen of said sleeve of film material (F), said sleeve of film material being closed at a bag bottom (BB),
- closing the receiving lumen at a bag head so as to form a bag (WB) when the desired amount of processed waste is within the receiving lumen.

7. **The** method according to Claim 6, comprising, prior to closing the receiving lumen at a bag head, operating the supply unit (9) of said negative pressure fluidic circuit in order to suck air away from said receiving lumen.

8. The method according to Claim 7, wherein the discharge opening (4) is kept open during said sucking of air away from the receiving lumen, the sucking of air away from the receiving lumen taking place through said processing volume (V2) and said discharge opening (5) .

9. The method according to any one of Claims 6 to 8, further comprising closing the receiving lumen at a bag bottom of a following bag, and separating the bag (WB) from the following bag by means of said separation device (26).

10. A method for the storage of waste processed in a processing machine (1), comprising:
- a processing cell (2) configured to receive waste to undergo processing into a processing volume (V2), the processing cell (2) comprising a main axis (Z2), a closable loading opening (4) arranged at a top end (2T) thereof, and a closable discharge opening (5) which is arranged at a bottom end (2B) thereof, the bottom end (2B) and the top end (2T) being opposite ends along said main axis (Z2),
- a rotor (7) arranged in the processing volume (V2) of said processing cell (2) at the bottom end (2B) thereof, the rotor (Z2) being operable in rotation around said main axis (Z2) and comprising cutting elements configured to heat and shred the waste in said processing volume (V2),
- a negative pressure fluidic circuit, in fluid communication (11) with the processing volume (V2), the fluidic circuit being controllable by a supply unit (9) configured for the establishment of a negative pressure within the processing volume (V2),
- an interface chamber (17) in communication with said discharge opening (5) and with a drop channel (19) configured to receive a bag for storing the waste processed by the machine (1),
the method comprising:
- coupling a bag for storing waste to said drop channel (19),
- enabling the communication between the processing volume (V2) and the drop channel(19) through the discharge opening (5),
- rotating the rotor (7) to direct a desired amount of processed waste through said discharge opening (5) and drop channel (19) into the waste storage bag,
- operating the supply unit (9) of said negative pressure fluidic circuit in order to suck air away from said waste storage bag,
- closing the waste storage bag.

11. A machine (1) for the processing of waste, including:
- a processing cell (2) configured to receive waste to undergo processing into a processing volume (V2) thereof, the processing cell (2) comprising a main axis (Z2), a closable loading opening (4) arranged at a top end (2T) thereof, and a closable discharge opening (5) arranged at a bottom end (2B) thereof, the bottom end (2B) and the top end (2T) being opposite ends along said main axis (Z2),
- a rotor (7) arranged in the processing volume (V2) of said processing cell (2) at the bottom end (2B) thereof, the rotor (7) being operable in rotation around said main axis (Z2) and comprising cutting elements configured for heating and shredding the waste in said processing volume (V2),
the machine (1) being **characterized in that** the processing volume (V2) comprises an inner diameter extending transversely to said main axis (Z2), said inner diameter decreasing along said main axis (Z2) towards said bottom end (2B).

12. The machine (1) according to Claim 11, wherein the inner diameter of the processing volume (V2) has a first value (DT) at said top end (2T) and a second value (DB) at said bottom end (2B), the inner diameter of the processing volume decreasing from said fist value (DT) to said second value (DB) along said main axis (Z2) from said top end (2T) to said bottom end (2) .

13. The machine (1) according to any one of Claims 11 and 12, wherein the loading opening (4) and the discharge opening (5) are hermetically closable,
the machine (1) moreover comprising a negative pressure fluidic circuit in fluid communication (11) with the processing volume (V2), the fluidic circuit being controllable via a supply unit (9) configured to establish a negative pressure within the processing volume (V2).

14. **The** machine (1) according to any Claim 11 to 13, wherein the discharge opening (4) is in communication (17, 19) with a storage unit (20), the storage unit (20) comprising:
- a annular support (21) configured to receive a sleeve of film material (F) partially accumulated in a collapsed state and being deployable to define a receiving lumen for the waste processed by machine (1), said receiving lumen being closable in order to define a bag (WB) for receiving the waste processed by the machine (1),
- a closing unit (23) comprising one or more closing devices (24, 25) configured to close the receiving lumen at a bag bottom and at a bag head, in order to define said bag (WB), and moreover comprising a separation device (26) configured to separate a bag head of a first bag (WB) from the bag bottom of a second bag (WB) following the first bag along the sleeve of film material (F).

15. Machine (1) according to Claim 14, including an interface chamber (17) in communication with said discharge opening (5) and with a drop channel(19) opening into the annular support (21) of the storage unit, so as to lead the waste coming from said discharge opening (5) into the receiving lumen of the sleeve of film material (F).
